# EUROPEAN PATENT APPLICATION

(11) **EP 0 655 245 A2**
(43) Date of publication of application: **31.05.1995**
(21) Application number: 94308005.1
(22) Date of filing: 31.10.1994
(51) Int. Cl.: A61K 31/425, A61K 38/00, A61K 38/06, A23L 1/30, A23L 1/305, A23L 1/302, A23L 1/304

(54) **Compositions and their use for retarding the aging process**

(30) Priority: 01.11.1993 US 146305
(71) Applicant: FREE RADICAL SCIENCES Inc, Cambridge Massachusetts 02139 (US)
(72) Inventor: Kamerei, Ahmad Reza, Wilmette, Illinois 60091 (US); Goldberg, Dennis I., Sudbury MA 01776 (US); Mark, David A., Illinois (US); Pace, Gary, Cambridge, Massachusetts 02142 (US)
(74) Representative: MacGregor, Gordon

(57) **Abstract**

Compositions, diets and regimens are disclosed for maintaining intracellular levels of glutathione at sufficient levels to prevent oxidative and free radical damage to the cells, so as to retard the aging process in mammals.

## Description

### SPECIFICATION

### BACKGROUND OF THE INVENTION

The present invention relates generally to the treatment of diseases and disorders. More specifically, the present invention relates to retarding the aging process.

Since the beginning of civilization, man has searched for ways to slow down the aging process. Although a variety of techniques, nutrients, and medicaments have been explored, the search for a method and/or composition for reducing the aging process has been elusive. One of the difficulties in developing useful methods is that there is very little uniformity in the scientific community regarding the mechanism of the aging process.

A popular theory on aging is the free radical theory. Pursuant to this theory, oxidative damage of cell organelles and components (DNA, RNA, protein, lipids, etc.) caused by lipid peroxidation, ionizing radiation, ultraviolet rays, and chemicals results in a loss of functionality, degregation, degeneration, and the death of cells.

A number of investigators have looked at the free radical mechanism of aging. *See, Harmon*, Free Radical Theory of Aging: Effect of the Amount and Degree of Unsaturation of Dietary Fat on Mortality Rate, *J. Gerontology* 26 (4) 451-457, 1971; *Shock et al,* Normal Human Aging: The Baltimore Longitudinal Study of Aging, NIH Publication No. 84-2450, U.S. Government Printing Office, Washington, D.C., 1984; *Ordy et al,* Nutrition in Gerontology (1984), Raven Press, New York; *Finch et al*, (1985) Handbook of the Biology of Aging, Second Edition, Van Nostrand Reinhold Company, New York; *Alberede et al*, (1991) Facts and Research in Gerontology, Serdi Publication, Paris *Mooradian*, (1990); Molecular Theories of Aging In: Geriatric Nutrition, A Comprehensive Review, Ed: J.E. Morley, Z. Glick, and L.Z. Rubenstein, Raven Press, New York; *Schneider et al*, (1985), Modulations of Aging Processes, In: Handbook of the Biology of Aging, Ed: C.E. Finch and E.L. Schneider, Van Nostrand Reinhold Co., New York; and *Yu* (1991) Free Radicals and Modulation by Dietary Restriction, *Age* & *Nutrition* 2 (2) 84-89.

The aging process in human beings and animals is a combination of extremely complex biochemical phenomena. There may be one or more mechanisms involved in the aging process (e.g., free radical reactions, Malliard reactions, damaged proteins synthesizing machinery, etc.). However, at this time, the state of the art does not appear able to stop these naturally occurring processes.

Senescence is especially defined as "an increased vulnerability occurring with advancing age." *Comfort*, The Biology of Senescence, 3rd Edition (1979). As part of the aging process, there is a progressive decline in T-cells function, as well as T-cells, B-cells, macrophages, and neutrophils becoming less responsive to up and down regulation. It is believed, at least by the inventors, that they also become more susceptible to free radical damage. As part of the aging process, this immune senescence occurs.

Similar to other aspects of aging, there is no uniformity of opinion on the consequence of immune senescence. For example, the immune system is thought to play an important role in preventing the growth and metastases of cancer cells. However, even though the incidence of most solid tumor cancers increase exponentially with age, there is a dispute whether there is any true correlation between aging and cancer. *See*, *Richie*, The Role of Glutathione in Aging and Cancer, Experimental Gerontology, Vol. 27, pp. 615-626 (1992) and discussion therein.

A vast number of ingredients and compounds have been cited for use to render some direct or indirect anti-aging effect in animals. These compositions include, nutrients: Vitamin C; Vitamin D; Vitamin E; Vitamin B6; Vitamin B12; beta-carotene; selenium; calcium; iron; and zinc. Unfamiliar nutrients have been used such as: limonoids; glucarates; phenolic acid; flavonoids; coumarines; polyacetylenes; carotenoids; and Vitamin PPQ.

Likewise, various compounds have been used such as: coenzyme Q10; RNA; glutathione; synthetic antioxidants (BHA, BHT, TBHQ); antioxidant PBN; glutamate; choline; and acetylcholine. Enzymes have also been used such as: SOD (superoxide) dismutase; and catalase.

Additionally, foods and certain ingredients have been explored such as: garlic; licorice root; flax seed; citrus fruits; umbelliferous vegetables (carrots, parsley, celery); ginseng; bee pollen; and royal jelly.

Likewise, certain hormones have been explored such as: human growth hormones; DHEA (dehydroepiandrosterone); estrogen; testosterone; thymosin; melatonin; aminoguanidine; and nerve growth factors.

Further, certain drugs have also been considered and used including: deprenyl; Gerovital H-3; hydergine; L-dopa; lypressin; dilantin; and Ginkgo-Biloba.

It is believed that none of these components alone provide an effective composition for slowing the aging process. There is therefore a need for a composition for slowing the aging process.

### SUMMARY OF THE INVENTION

The present invention provides methods and compositions for retarding the aging process in mammals. Pursuant to the present invention, intracellular levels of glutathione are maintained at sufficient levels to prevent oxidative and free radical damage to the cells.

In an embodiment of the present invention, a composition that stimulates the intracellular synthesis of glutathione is administered to elevate the levels of intracellular glutathione in a mammal to sufficient levels or to maintain sufficient intracellular glutathione levels. The composition can be administered either parenterally (intravenously, intramuscularly, intraperitoneally) or enterally (tablets, pills, capsules, syrup, or through diet).

To this end, the present invention provides a method for retarding the aging process in a mammal comprising the steps of administering to the mammal on a regular basis, a biologically effective amount of a composition that stimulates the intracellular synthesis of glutathione to maintain in the mammal a sufficient level of glutathione to at least reduce oxidative damage to cells caused by free radicals.

In an embodiment, the composition includes at least one stimulator of intracellular glutathione synthesis chosen from the group consisting of: L-2-oxothiazolidine-4-carboxylate; esters of L-2-oxothiazolidine-4-carboxylate; glutathione esters; and proteins rich in or enriched with cysteine.

In an embodiment, the composition is administered at least daily.

In an embodiment, the sufficient level of glutathione in circulating lymphocytes (as an exemplary indicator) is at least 7-8 nmol/mg protein.

In an embodiment, the method includes the steps of providing the composition by regulating the dietary intake of the person to insure sufficient cysteine rich proteins are ingested and supplementing, as necessary, the patient's diet with a compound chosen from the group consisting of: L-2-oxothiazolidine-4-carboxylate; esters of L-2-oxothiazolidine-4-carboxylate; and glutathione esters.

In an embodiment, the method includes the step of monitoring the glutathione levels of the mammal.

In an embodiment of the method, the mammal's glutathione levels are at least maintained at greater than normal levels.

In a further embodiment, the present invention provides a method for retarding the aging process in a person comprising the step of administering to a person a diet including: approximately 15% to about 30% of the calories from whey protein or a protein and peptide mixture with equivalent or greater cysteine content; approximately 15% to about 25% of the calories from a lipid source; approximately 45% to about 70% of the calories from carbohydrates; and a vitamin/mineral mixture that meets or exceeds the U.S. RDA values in 1,000 to 2,000 calories of product.

In an embodiment, the method includes the step of additionally administering to the patient a biologically effective amount of a composition chosen from the group consisting of: L-2-oxothiazolidine-4-carboxylate; esters of L-2-oxothiazolidine-4-carboxylate; and glutathione esters.

The present invention also provides a diet for retarding the aging process in a person comprising, in an embodiment: approximately 15-30% of the calories from whey protein or peptide mixture with equivalent or greater cysteine content; approximately 15-25% of the calories from lipids; approximately 45-70% of the calories from carbohydrates; and a vitamin/mineral mixture meeting or exceeding U.S. RDA values in 1,000 to 2,000 calories of product. The diet, if desired or necessary, can be supplemented with a composition such as: L-2-oxothiazolidine-4-carboxylate; esters of L-2-oxothiazolidine-4-carboxylate; or glutathione esters.

An advantage of the present invention is that it provides a composition that can retard the aging process in mammals.

Still further, an advantage of the present invention is to provide a method for reducing the aging process in humans.

Moreover, an advantage of the present invention is that it provides a method for retarding the aging process that can be given enterally and even by controlling the diet of a person.

Furthermore, an advantage of the present invention is that it provides a diet for retarding the aging process that does not necessarily produce weight loss.

Further, an advantage of the present invention is to provide a method for retarding the aging process that can be based principally on a diet and supplemented as necessary with a medicament.

Additional features and advantages of the present invention are described in, and will be apparent from, the detailed description of the presently preferred embodiments and from the drawings.

### DETAILED DESCRIPTION OF THE PRESENTLY PREFERRED EMBODIMENTS

The present invention provides compositions and methods for retarding the aging process in mammals. Pursuant to the present invention, compositions that stimulate the intracellular synthesis of glutathione are administered to a mammal.

Glutathione has multiple physiological and metabolic functions. These functions include: thiol transfer reactions (e.g., thio-disulfide reactions); a reducing capacity for several reactions (e.g., formation of deoxyribonucleotides from ribonucleotides); destruction of hydrogen peroxide, organic peroxides, free radicals, and certain foreign compounds; chemical participation (as catalysts or reactants in metabolism of various endogenous compounds; and transport of amino acids (possibly certain amines and peptides).

Pursuant to the present invention, the intracellular synthesis of glutathione is optimized to insure that sufficient glutathione is present. At a minimum, the intracellular levels are stimulated to insure that at least normal levels of intracellular glutathione are produced intracellularly. Due to stress and disease states, as well as the aging process, intracellular levels of glutathione can become reduced. Thus, the cells become vulnerable to the damaging effects of free radicals and oxidative compounds that can be part of the aging process.

By using intracellular glutathione stimulators, the intracellular concentration of glutathione can be maintained at sufficiently high levels to provide a protective function to the cells. Different types of cells may require different levels of glutathione. For example, ideally, the intracellular glutathione levels should be maintained at approximately 8 to about 10 nmol/mg protein in circulating lymphocytes.

Although glutathione is a naturally-occurring compound that could be externally provided to the body, there are difficulties in its administration. *Witshi et al*, The Systemic Availability of Oral Glutathione, Eur. J. Clin. Pharmacol (1992) 43, 667-69, reports that it is not possible to increase circulating glutathione to a clinically beneficial extent by the oral administration of a single dose of 3 g glutathione in a healthy human. Cysteine is the rate-limiting amino acid for intracellular glutathione synthesis. Although it may be used at low to moderate concentrations, at high concentration levels, cysteine may show some toxicity. "Increasing dietary levels of the GSH precursors cysteine and methionine do not appear to offer a practical intervention due to their toxicity." *Furukawa et al*, The Potential Benefits of Dietary Glutathione on Immune Function and Other Practical Implications, Glutathione: Metabolism and Physiological Function, pp. 351-355 (1990). Furthermore, cysteine is unstable in solutions.

L-2-oxothiazolidine-4-carboxylate, glutathione esters, and esters of L-2-oxothiazolidine-4-carboxylate are stable non-toxic cysteine sources which are readily bioavailable. These compositions can be readily utilized via various administration routes including parenteral and enteral.

L-2-oxothiazolidine-4-carboxylate is subjected to the action of 5-oxo-L-prolinase in the presence of adenosine triphosphate to produce S-carboxyl cysteine. S-carboxyl cysteine is then decarboxylated to produce cysteine. Cysteine is then metabolized to provide glutathione. See, U.S. Patent Nos.: 4,335,210; 4,434,158; 4,438,124; 4,665,082; and 4,647,571, the disclosures of which are incorporated herein by reference.

Likewise, esters of L-2-oxothiazolidine-4-carboxylate can be used. The ester can be saturated straight or branched, alkyl group of 1 to 10 carbon atoms. Preferably, the ester is chosen from a saturated straight alkyl group such as methyl, ethyl, propyl, butyl, pentyl, hexyl, heptyl, octyl, nonyl, or decyl and a saturated branched alkyl group such as isopropyl, isobutyl, sec-butyl, tert-butyl, or isopentyl. Such esters are disclosed in U.S. Patent 5,208,249, the disclosure of which is hereby incorporated herein by reference.

Other substrates that optimize intracellular glutathione synthesis can also be utilized to retard the aging process. To this end, glutathione esters, as well as other thiazolidine-4-carboxylate analogs, that are converted intracellularly to glutathione can be utilized.

Such a glutathione ester, for example, can have the structure: 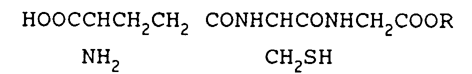
wherein: R is an alkyl group containing 1 to 10 carbon atoms. Preferably, the methyl and ethyl glutathione esters are used. Glutathione esters are disclosed in U.S. Patent No. 4,710,489, the disclosure of which is incorporated herein by reference.

Further, proteins rich in cysteine, that will also optimize intracellular glutathione synthesis, can be utilized pursuant to the present invention to reduce the aging process. Such proteins, if they have sufficiently high cysteine levels and are administered in sufficiently great quantities, it is believed, can provide intracellular glutathione stimulation. For example, the following proteins have a high cysteine content: whey (2.3%); egg white (2.5%); serum albumin (5.5%); and lactalbumin (5.8%). It is envisioned that such proteins can be used either alone as part of a diet, or if and when necessary in combination with L-2-oxothiazolidine-4-carboxylate, esters of L-2-oxothiazolidine-4-carboxylate, and/or glutathione esters.

Pursuant to the present invention, the compositions of the present invention can either be administered parenterally or enterally. Enterally, as set forth in detail below, the composition can comprise, at least in part, a diet including proteins rich in cysteine. However, when necessary, the diet can be supplemented with, for example, a pill including, for example, L-2-oxothiazolidine-4-carboxylate, that is administered to a patient.

Parenterally, the composition can comprise, in part, a solution that comprises approximately 3% L-2-oxothiazolidine-4-carboxylate in a physiologic (e.g., phosphate) buffer. The composition can be administered either intravenously or through another route, such as intraperitoneally.

It is envisioned that the present invention would be used prophylactically. In this regard, pursuant to a method of the present invention, a person would be placed on a regimen that would provide sufficient intracellular glutathione stimulation in order to insure that on a daily basis, the cells have sufficient levels of glutathione. However, during periods of stress and disease states, additional intracellular glutathione stimulation can be provided either through an enteral or parenteral method using: L-2-oxothiazolidine-4-carboxylate; and/or glutathione ester. Likewise, as the person gets older additional intracellular glutathione synthesis can be provided through these compounds to deal with naturally occurring reductions.

For example, the patient can be placed on a diet with sufficient proteins rich in cysteine in order to insure sufficient intracellular glutathione level. By way of example, and not limitation, an example of the anti-aging diet of the present invention is as follows:
Liquid, ready-to-use enteral product suitable for oral use or tube-feeding, with a caloric density of 1.0-1.5 kcal/mL -
15-30% of calories from whey protein, or protein source or peptide mixture with equivalent or greater cysteine content (≧ 2% of protein);
15-25% of calories from lipids;
45-70% of calories from carbohydrates;
vitamin/mineral mixture meeting or exceeding U.S. RDA values in 1,000 to 2,000 calories of product;
Beta-carotene ≧ 3000 IU/1,000 calories;
Vitamin C ≧ 150 mg/1,000 calories;
Vitamin E ≧ 45 mg/1000 calories;
Zinc ≧ 20 mg/1,000 calories; and
Selenium ≧ 150 micrograms/1,000 calories.

The diet, unlike calorie restricted diets, provides sufficient calories via high quality proteins, balanced lipids, various carbohydrates, and adequate vitamins and minerals. The diet also contains anti-aging compounds.

It should be noted that *Schneider* and *Reed*, supra, found that many of the interventions that have been reported to increase life span produce weight loss. It has been noted that the only nutritional manipulation that has been reproducibly found to increase the life span of mammalian species is that of food restriction in rodents. See, *Masoro* (1990), Nutrition and Longevity, IN Geriatric Nutrition, Ed.: J.E. Morley, Z. Glick, and L.Z. Rubenstein, Raven Press, New York. *Masoro* postulated that food restriction may influence the aging process by protecting the animal from free radical damage or by modulating age-related changes in gene expression.

However, in contrast, the diet of the present invention does not, by necessity result in weight loss. Therefore, the diet is more acceptable and versatile and should be more acceptable to a large percent of the population.

Pursuant to the present invention, the patient's blood levels, who is receiving the diet, can be monitored to determine circulating blood levels of glutathione. During stress states or when circulating blood levels of glutathione drop below a certain level, e.g. 1.2 micromol/ml additional intracellular glutathione stimulation can be provided.

To this end, for example, such intracellular glutathione stimulation can be provided either by enterally administering pills containing L-2-oxothiazolidine-4-carboxylate, esters of L-2-oxothiazolidine-4-carboxylate, and/or glutathione esters or through a bolus injection of L-2-oxothiazolidine-4-carboxylate, esters of L-2-oxothiazolidine-4-carboxylate, and/or glutathione esters. In this way, the aging process caused by cell damage due to free radicals and other oxidative processes can be reduced and the aging process slowed down.

By way of example, and not limitation, a contemplative example of the present invention will now be given.

### EXAMPLE NO. 1

Two hundred and twenty men and women with an average age of 30±5 years are recruited for a study. They are assessed for nutritional status, and found, on the average, to be marginally protein-calorie malnourished and at low or deficient levels for vitamin A, beta-carotene, vitamin C, vitamin E, zinc and selenium. Immunological status is assessed through use of delayed-type hypersensitivity (DTH) skin response, and in vitro analyses: white blood cell count, mitogenic response to concanavalin A (Con A), IL-2 production and blood and intracellular glutathione measurements. Responses are low compared to published values for healthy adults ages 20-40 and blood and intracellular GSH levels are 20-40% below normal. Furthermore, the in vitro addition of the sulphur-donating compound, 2-mercaptoethanol (2-ME), to Con A cultures results in a greater increase in the mitogenic response than typically seen with 2-ME and cells from younger people.

Half of the population is given 50-80% of their daily caloric needs in the form of an oral nutritional supplement with a higher cysteine content, a low omega-6 fatty acid content and increased amounts of key vitamins and minerals as described earlier. The remainder of their diet is the standard diet. The people in the other half of the study receive the standard nursing home diet. At the end of six months all patients are tested again.

Blood and intracellular GSH concentrations were increased in the supplemented group, as were vitamins A, C, and E and beta-carotene. Zinc and selenium levels were increased. The treated group showed increases in Con A, DTH and IL-2 responses. In response to 2-ME, the percent increase in mitogenic response in the supplemented group was lower than the control group, suggesting that the former now had adequate cysteine and GSH tissue levels. Furthermore, the in vivo antibody response to influenza vaccine was higher in the supplemented group, and over the course of the six months they had fewer infectious illnesses and fewer courses of treatment with antibiotics.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. Use in a preparation for the purpose of retarding the aging process in a mammal of a composition that stimulates the intracellular synthesis of glutathione in an amount to maintain in the mammal at least 5 nmol/mg protein level of glutathione in circulating lymphocytes.

2. Use according to Claim 1 wherein said amount is sufficient to maintain the protein level at at least 7 nmol/mg.

3. Use according to Claim 1 or 2 wherein the composition includes at least one component for stimulating the intracellular synthesis of glutathione chosen from: L-2-oxothiazolidine-4-carboxylate; esters of L-2-oxothiazolidine-4-carboxylate; glutathione esters; and proteins or peptides rich in cysteine.

4. Use according to Claim 3 including proteins rich in cysteine in addition to at least one of L-2-oxothiazolidine-4-carboxylate; esters of L-2-oxothiazolidine-4-carboxylate; and glutathione esters.

5. Use according to Claim 3 or 4 wherein the proteins rich in cysteine have a cysteine content of at least 2% by weight of the protein.

6. Use according to Claim 4 or 5, wherein the cysteine-rich protein includes whey protein, egg white, lactalbumin and/or serum albumin.

7. Use according to any preceding claim wherein the composition is enterally administrable.

8. Use according to any one of Claims 1 to 6 wherein the composition is parenterally administrable.

9. A diet, regimen or nutritional composition for reducing aging in a person comprising:
15 to 30% of the calories from cysteine-rich protein;
15 to 25% of the calories from lipids;
45 to 70% of the calories from carbohydrates; and
vitamin/mineral mixture meeting or exceeding US RDA values in 1,000 or 2,000 calories of product.

10. The diet, regimen or composition of Claim 9, including beta-carotene ≧ 3,000 IU/1,000 calories, Vitamin C ≧ 150 mg/1,000 calories, Vitamin E ≧ 45 mg/1,000 calories, zinc ≧ 20 mg/1,000 calories, and selenium ≧ 150 micrograms/1,000 calories.

11. The diet, regimen or composition of Claim 9 or 10 including at least one component chosen from L-2-oxothiazolidine-4-carboxylate; esters of L-2-oxothiazolidine-4-carboxylate; and glutathione esters.

12. The diet, regimen or composition of Claim 9, 10 or 11, wherein the cysteine-rich protein includes whey protein, egg white, lactalbumin and/or serum albumin.

13. Use according to any one of Claims 1 to 8, wherein the composition is according to Claim 11.
